# FASCICULE DE BREVET EUROPEEN

(11) **EP 0 596 799 B1**
(45) Date de publication et mention de la délivrance du brevet: **06.03.1996**
(21) Numéro de dépôt: 93402699.8
(22) Date de dépôt: 03.11.1993
(51) Int. Cl.: C07C 5/333

(54) **Procédé et dispositif de déshydrogénation catalytique d'une charge paraffinique C2+ comprenant un système d'auto-réfrigération**
Verfahren und Vorrichtung zur katalytischen Dehydrierung von paraffinischen C2+ Beschickung, mit Selbstkühlung
Process and apparatus for the catalytic deshydrogenation of a paraffinic C2+ feed comprising an auto-refrigeration system

(30) Priorité: 06.11.1992 FR 9213515
(43) Date de publication de la demande: 11.05.1994
(73) Titulaire: INSTITUT FRANCAIS DU PETROLE, F-92502 Rueil-Malmaison (FR)
(72) Inventeur: Minkkinen, Ari, F-78860 Saint Nom la Breteche (FR); Burzynski, Jean-Pierre, F-69110 Sainte-Foy-les-Lyon (FR)

(56) Documents cités:
- US-A- 4 381 417
- US-A- 4 381 418
- US-A- 4 663 493

## Description

L'invention concerne un procédé de déshydrogénation catalytique d'une charge hydrocarbonée paraffinique C₂+ et un dispositif de mise en oeuvre du procédé. Elle a trait plus spécialement à la séparation d'un gaz riche en hydrogène et d'une phase liquide hydrocarbonée à partir d'effluents de réaction à basse pression contenant de l'hydrogène destiné à être recyclé.

L'art antérieur est illustré par les brevets US-A-4 381 417, US-A-4 381 418 et US-A-4 663 493.

Il est connu qu'un nombre de procédés industriels mettant en oeuvre les réactions catalytiques à basse pression opèrent dans un environnement d'hydrogène dans lequel la pression partielle en hydrogène est assurée par un recyclage d'un gaz riche en hydrogène contenu dans un effluent réactionnel et qui a été séparé des hydrocarbures.

C'est le cas notamment d'un procédé de déshydrogénation catalytique des GPL contenant du propane, du butane et des isobutanes pour produire des monooléfines qui servent d'intermédiaire pour les carburants à haut indice d'octane. Dans le cas de la déshydrogénation de l'isobutane, l'isobutène produit peut réagir avec du méthanol pour produire du méthyltertiobutyléther, un additif utilisable dans les essences.

L'art antérieur est aussi illustré par les brevets US 4 381 418 et US 4 381 417. Dans de tels procédés la réaction est réalisée dans un réacteur catalytique continuellement régénéré opérant à très faible pression (légèrement supérieure à la pression atmosphérique ou sous vide) et à des températures de 500 à 600°C.

L'hydrogène recyclé et l'hydrogène produit assurent une pression partielle en hydrogène suffisante pour inhiber la formation de coke et de ce fait maintenir la stabilité du catalyseur. On atteint ainsi une conversion satisfaisante à une gamme de températures plus élevée, arrivant par exemple à 600°C. En règle générale, l'effluent à basse pression délivré par la zone réactionnelle de déshydrogénation est refroidie d'abord par échange de chaleur avec la charge gazeuse puis avec de l'eau, à une température appropriée avant que la pression de vapeur de l'effluent ne soit efficacement remontée dans un équipement de compression conventionnel, à une pression supérieure, ce qui permet la séparation de l'hydrogène et des composés hydrocarbonés de l'effluent.

La séparation de l'hydrogène des hydrocarbures de l'effluent se réalise en général à une pression supérieure à celle régnant dans la zone réactionnelle. De plus, pour condenser les hydrocarbures dans le mélange gazeux constituant l'effluent et contenant l'hydrogène, il est nécessaire de refroidir à une température inférieure à celle que peuvent atteindre les échangeurs de chaleur à l'air ou à l'eau traditionnels. Ceux-ci sont par ailleurs encombrants.

D'autres systèmes de réfrigération externes préconisent des cycles au propane ou au propène mais ils sont chers et grands consommateurs d'énergie.

Par ailleurs, puisqu'une réfrigération en-dessous de 0°C est requise pour la séparation de l'hydrogène et des composés hydrocarbonés, l'eau présente dans l'effluent comprimé doit être éliminée et atteindre une concentration limite telle qu'il n'y ait pas congélation, de façon à prévenir l'obstruction de l'équipement de réfrigération. On utilise à cette fin des tamis moléculaire 3 Å (1 Å = 1 x 10⁻¹⁰ m) pour déplacer l'eau de l'effluent avant l'étape de réfrigération.

Un objet de l'invention est de remédier aux inconvénients mentionnés ci-dessus et plus particulièrement de parvenir à une séparation aussi complète que possible entre l'hydrogène de recyclage et les composés hydrocarbonés oléfiniques de l'effluent de façon à atteindre un taux optimal de conversion.

Un autre objet de l'invention est de proposer un circuit de réfrigération de l'effluent comprimé complètement autoréfrigéré éliminant la nécessité de cycles de réfrigération externes.

Un autre objet de l'invention est de proposer un réacteur fonctionnant sous une pression la plus faible possible en relation directe avec des équipements en aval à faible perte de charge établissant une contre-pression la plus faible possible, ce qui contribue à augmenter le taux de conversion de l'unité.

De manière générale, l'invention concerne un procédé de déshydrogénation catalytique d'une charge hydrocarbonée paraffinique C₂ ⁺ comprenant une étape de déshydrogénation dans une zone de déshydrogénation, de ladite charge en phase gazeuse éventuellement en présence d'hydrogène délivrant un effluent de déshydrogénation comprenant de l'eau, de l'hydrogène, des hydrocarbures oléfiniques et des hydrocarbures paraffiniques non convertis, au moins une étape de refroidissement de l'effluent dans une zone de refroidissement indirect avec ladite charge, une étape de compression de l'effluent à une pression appropriée, une étape d'élimination de l'eau de l'effluent comprimé, une étape de réfrigération dans un échangeur de chaleur à double enceinte de l'effluent comprimé, une étape de séparation de l'effluent réfrigéré et une étape de récupération d'une part d'une phase vapeur riche en hydrogène et d'autre part d'une phase liquide hydrocarbonée comprenant les hydrocarbures oléfiniques et les hydrocarbures non convertis.

Plus précisément, I'étape de réfrigération comprend l'introduction dans une première enceinte dudit échangeur de chaleur, de la charge hydrocarbonée en phase liquide, I'échangeur de chaleur étant adapté à vaporiser ladite charge dans la première enceinte et à réfrigérer par échange indirect l'effluent comprimé dans la seconde enceinte, le procédé étant en outre caractérisé en ce qu'on récupère de la première enceinte de l'échangeur de chaleur la charge vaporisée que l'on introduit dans la zone de déshydrogénation.

L'effluent avant d'être réfrigéré est généralement comprimé à une pression adéquate pour pouvoir récupérer lors des étapes de réfrigération et de séparation au moins 95 % et de préférence au moins 98 % d'isobutène dans l'effluent, s'il s'agit d'une coupe C₄.

Selon une première variante du procédé, on peut recycler une partie au moins de la phase riche en hydrogène dans la première enceinte de l'échangeur de chaleur où on la met en contact avec la charge liquide et on récupère la charge vaporisée contenant l'hydrogène que l'on introduit dans la zone de déshydrogénation.

Selon une autre variante du procédé, on peut détendre de manière isentropique par des moyens de détente appropriés la phase vapeur riche en hydrogène de façon à séparer un condensat résiduel hydrocarboné et de l'hydrogène, on peut recycler en partie au moins l'hydrogène ainsi séparé dans la première enceinte de l'échangeur de chaleur où on le met en contact avec la charge liquide et on peut récupèrer la charge vaporisée et l'hydrogène que l'on introduit dans la zone de déshydrogénation.

Selon une caractéristique de cette dernière variante, le condensat résiduel hydrocarboné résultant de la séparation à basse pression de la phase vapeur riche en hydrogène et donc à température très basse peut être mélangé à l'effluent de la seconde enceinte de l'échangeur de chaleur pour abaisser sa température de quelques degrés supplémentaires (2 à 10°C par exemple).

Selon une autre caractéristique du procédé, on peut avantageusement abaisser la température de l'effluent dans l'échangeur de chaleur en comprimant la charge vaporisée avec ou sans l'hydrogène recyclé sortant de la première enceinte de l'échangeur de chaleur. On abaisse de ce fait la pression à l'intérieur de la première enceinte de l'échangeur de chaleur à 0,2 à 3 bar absolus. Cette étape d'abaissement de pression dans la première enceinte combinée avec la réfrigération par la charge vaporisée et l'hydrogène est d'autant plus avantageuse que la charge contient des hydrocarbures lourds, par exemple des hydrocarbures en C₅.

Par ailleurs, ces étapes de réfrigération supplémentaire permettent d'éviter des niveaux de compression élevés de l'effluent en amont de l'étape d'élimination de l'eau et donc de minimiser les coûts en investissement et en fonctionnement. Il est donc possible de combiner avantageusement les niveaux de pression, de température ainsi que la quantité d'hydrogène recyclé dans la première enceinte de l'échangeur thermique pour obtenir au plus bas coût possible la température de séparation des phases.

En règle générale, la charge peut comprendre des hydrocarbures paraffiniques à 3, 4 et/ou 5 atomes de carbone. Plus particulièrement, elle peut comprendre de l'isobutane. Elle peut, par ailleurs, être constituée d'une charge fraiche et d'hydrocarbures paraffiniques non convertis recyclés.

Les conditions opératoires du procédé sont généralement les suivantes :
· Etape de déshydrogénation :
   Vitesse spatiale volumique (par rapport à la charge liquide) = 0,5 à 20 h⁻¹ de préférence 1,5 à 6 h⁻¹.
   P = 0,1 à 10 bar absolus, de préférence 1 à 4 bar. (1 bar = 10⁵Pa)
   T = 400 - 800°C, de préférence 500-600°C.
· Etape de refroidissement de l'effluent en sortie du réacteur par la charge vaporisée avec ou sans hydrogène
   T° = 50 - 150°C, de préférence 90 - 110°C.
· Etape de compression de l'effluent :
   P = 3 à 35 bar absolus et de préférence 10 à 18 bar.
   Température en sortie, de préférence 100-150°C.
· Etape de réfrigération : sans compression en aval.
   - enceinte du fluide froid (1ère enceinte)
      P = 1,2 - 5 bar absolus, de préférence 1,5 - 2,5 bar
      T = -5 à -90°C, de préférence -15 à -40°C.
      Rapport molaire H₂ sur charge = 0 - 5, de préférence 0,5 - 2.
   - enceinte de l'effluent (2ème enceinte)
      P = 3 - 35 bar, de préférence 8 à 12 bar absolus
      T = 0 à -85°C; de préférence -20 à -40°C.
· Etape de séparation des hydrocarbures de l'effluent, de la phase vapeur riche en hydrogène :
   P = 3 - 35 bar, de préférence 8 - 12 bar absolus
   T = 0 à -100°C, de préférence -20 à -40°C
· Etape de détente isentropique de la phase vapeur riche en hydrogène :
   P = 1,2 à 5 bar absolus, de préférence 2 à 3 bar
   T = -50 à -100°C, de préférence -75 à -85°C.
· Etape de compression de la charge vaporisée en aval de l'enceinte froide de l'échangeur de chaleur:
   Pression dans l'enceinte froide 0,2 à 3 bar absolus
   Température de la charge en sortie du compresseur 0 - 50°C, de préférence 10 à 20°C.

Selon une autre caractéristique du procédé, on peut comprimer l'autre partie de l'hydrogène qui a été détendu et séparé de la phase vapeur riche en hydrogène à une pression de 5 à 10 bar absolus délivrée par un compresseur mis en mouvement par les moyens de détente de la phase vapeur riche en hydrogène.

L'invention concerne aussi une unité de déshydrogénation catalytique comprenant en combinaison un réacteur de déshydrogénation (40), des moyens d'alimentation en une charge gazeuse hydrocarbonée et éventuellement des moyens d'alimentation en hydrogène, connectés à une entrée de réacteur (40), au moins un moyen (41) de refroidissement d'un effluent relié à une sortie du réacteur, des moyens de compression (6) de l'effluent connectés aux moyens de refroidissement, des moyens d'élimination (10) de l'eau contenue dans l'effluent connectés aux moyens de compression, des moyens de réfrigération (13) de l'effluent comprimé reliés aux moyens d'élimination de l'eau, des moyens de séparation (8) de l'effluent réfrigéré reliés aux moyens de réfrigération, des moyens de récupération d'une phase riche en hydrogène (22) et des moyens de récupération d'une phase riche en hydrocarbures oléfiniques (18), ladite unité étant caractérisée en ce que les moyens de réfrigération comprennent un échangeur (13) indirect de chaleur à double enceinte, une première enceinte ayant une entrée reliée aux moyens d'alimentation (12) en la charge liquide et une sortie reliée aux moyens de refroidissement (41), ledit échangeur (13) de chaleur étant adapté à vaporiser la charge dans ladite première enceinte, à l'évacuer par ladite sortie et à réfrigérer l'effluent par échange indirect de chaleur dans la seconde enceinte, ladite seconde enceinte étant reliée aux moyens de séparation (8).

Selon une première variante de l'unité, la première enceinte de l'échangeur de chaleur (13) comporte une autre entrée connectée aux moyens de récupération d'une phase riche en hydrogène (22) et des moyens adaptés à mélanger l'hydrogène et la charge.

Selon une deuxième variante de l'unité, les moyens de récupération d'une phase riche en hydrogène (22) comprennent des moyens de détente (23) connectés à un séparateur (27) de phases, ledit séparateur de phases ayant une première sortie de gaz hydrogène reliée à l'autre entrée de la première enceinte de l'échangeur de chaleur.

Par le procédé selon l'invention, on a obtenu d'excellents résultats en termes de séparation et de conversion. L'utilisation de la charge liquide comme source de réfrigération éventuellement au contact de l'hydrogène élimine le besoin d'échangeurs de chaleur externes, ce qui rend le procédé économique sur les plans des investissements, de l'énergie utilisée et des matériaux nécessaires dans les lignes de transfert, et ce qui réduit la contre-pression du système.

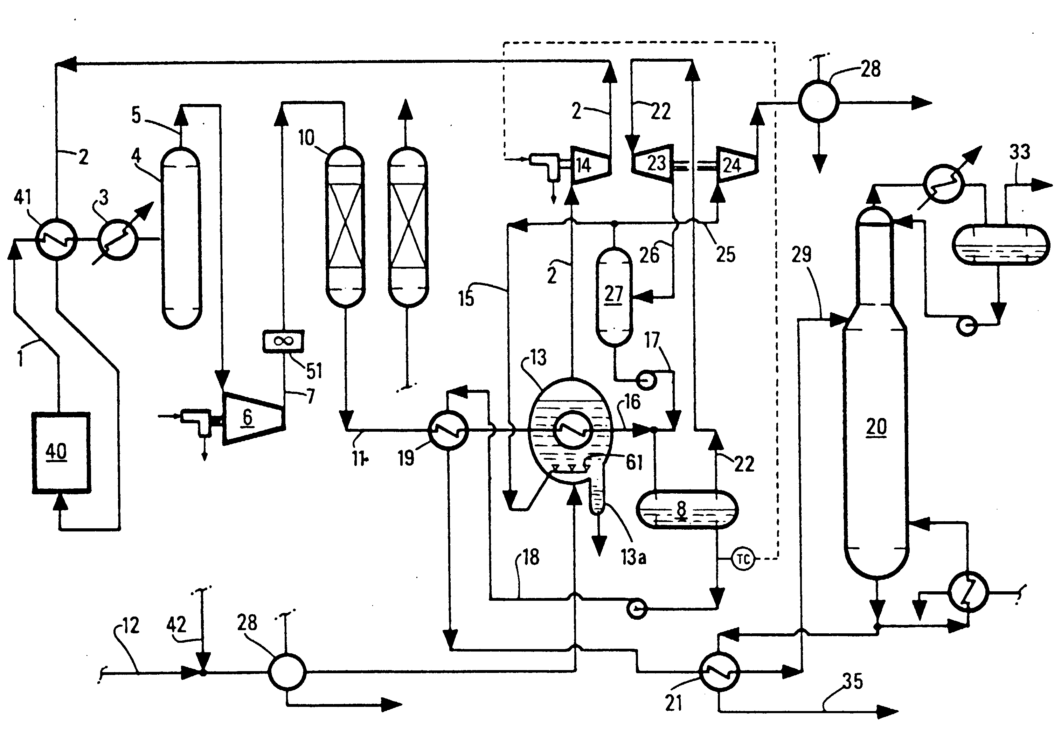

L'invention sera mieux comprise à la vue de la figure illustrant de façon schématique un mode de réalisation du procédé de déshydrogénation d'une coupe contenant de l'isobutane à 93 %.

La charge isobutane 2, sous forme gazeuse et de l'hydrogène, après avoir été préchauffés à une température appropriée par échange indirect dans un échangeur de chaleur à plaques 41, à faible perte de charge, sont introduits dans un réacteur 40 de déshydrogénation catalytique fonctionnant à basse pression (1,5 à 1,8 bar absolus) et à haute température 580-600°C. L'effluent qui en sort par la ligne 1 est refroidi par échange indirect dans cet échangeur à plaques, puis dans un autre échangeur 3 à faible perte de charge utilisant de l'eau comme fluide de refroidissement. L'effluent refroidi mais encore surchauffé après être passé dans un ballon de garde 4 alimente par une ligne 5 l'aspiration d'un système de compression 6 centrifuge à une pression sensiblement supérieure à la pression atmosphérique.

Le système de compression 6 élève la pression de l'effluent réactionnel à une valeur telle qu'une séparation efficace d'une phase hydrocarbonée liquide d'une phase gazeuse riche en hydrogène devient possible à une température généralement inférieure à 0°C. Dans le cas de la déshydrogénation de l'isobutane, une pression de 13 à 18 bar est tout à fait appropriée. Les besoins en énergie du système de compression peuvent être fournis par une turbine à gaz, une turbine à vapeur ou un moteur électrique.

Une ligne 7 est connectée à la sortie du compresseur 6 et conduit l'effluent comprimé puis refroidi dans l'échangeur 51 à travers au moins un lit 10 de tamis moléculaire 3 Å (1 Å = 1 x 10⁻¹⁰ m) pour enlever sensiblement toute l'eau et qui fonctionne selon un mode cyclique d'adsorption et de régénération. Une ligne 11 récupère en fond du lit l'effluent comprimé et déshydraté et le conduit dans un premier échangeur 19 de chaleur puis dans un second échangeur 13 de chaleur à tubes et à calandre adapté à réfrigérer l'effluent par échange indirect.

En effet, la charge d'isobutane fraîche et non convertie donc recyclée est amenée par une ligne 12 en phase liquide dans la calandre de l'échangeur 13. La présence d'eau peut être inhibée par ajout de méthanol 42 dans la ligne 12.

Cette phase liquide est vaporisée par la chaleur cédée par les tubes en présence d'une partie au moins d'hydrogène recyclé amené par une ligne 15 qui contribue à abaisser sa température d'ébullition à une pression donnée. Cet hydrogène est introduit à la partie inférieure de la calandre par des moyens 61, des tubes perforés par exemple, adaptés à favoriser le contact avec ladite phase liquide sensiblement dans tout le volume occupé par celle-ci. Elle sert dans ces conditions de fluide réfrigérant. Une phase gazeuse est récupérée dans la partie supérieure de la calandre, comprenant l'isobutane vaporisé et l'hydrogène recyclé et évacuée par une ligne 2 au moyen d'un compresseur 14 en direction de l'échangeur de chaleur à plaques 41 de façon à refroidir l'effluent puis en direction du réacteur 40.

On peut récupérer dans la partie inférieure 13a de la calandre un condensat de méthanol et d'eau qui peut être distillé dans l'unité de synthèse de MTBE, en aval.

La pression à l'intérieur de la calandre de l'échangeur de chaleur 13 adapté à réfrigérer l'effluent et donc la pression de vaporisation est maintenue à un niveau approprié par le compresseur 14 à simple étage et à vitesse variable qui a son entrée connectée directement à la calandre.

Pour obtenir une gamme avantageuse de températures de l'effluent de -25°C à -10°C, la pression dans la calandre est maintenue à environ 2 bar absolus. Par l'intermédiaire de ce compresseur 14, la charge en phase vapeur (isobutane et hydrogène) est comprimée vers l'entrée du réacteur de déshydrogénation à une pression de 3 à 4 bar absolus.

L'effluent réfrigéré dans les tubes de l'échangeur de chaleur 13 sort par une ligne 16 à une température d'environ -20 à -25°C par exemple et est dirigé vers un séparateur 8 de phases.

En tête du séparateur, une ligne 22 récupère une phase vapeur contenant de l'hydrogène en majeure partie et une faible proportion d'hydrocarbures et la conduit dans un turbodétendeur 23 où elle est détendue à entropie constante d'une pression par exemple de 15 bar à une pression d'environ 2,5 bar. A la sortie du turbodétendeur 23, la température dans la ligne 26 chute typiquement, de -25°C environ à -85°C, ce qui provoque la condensation d'une phase liquide hydrocarbonée dite cryogénique qui est séparée de l'hydrogène dans une enceinte de séparation 27. Cette phase liquide est récupérée en fond de l'enceinte 27 par une ligne 17 et mélangée à l'effluent réfrigéré sortant de l'échangeur de chaleur 13 réfrigérant, en un point en amont de l'entrée du séparateur 8. Ce contact direct éventuellement favorisé par des moyens de mélange appropriés contribue à refroidir par échange direct de chaleur l'effluent comprimé d'environ 2 à 10°C supplémentaires.

A la partie supérieure de l'enceinte de séparation 27, on récupère une phase gazeuse d'hydrogène (plus de 98 % en moles d'hydrogène) qui est divisée pour former un courant d'hydrogène destiné à être recyclé en partie par une ligne 15 et envoyé tout d'abord dans la calandre de l'échangeur de chaleur 13 réfrigérant pour vaporiser la charge liquide d'isobutane. L'autre partie du courant d'hydrogène est dirigée par une ligne 25 vers un compresseur 24 mis en mouvement par le turbodétendeur 23. Ce courant d'hydrogène comprimé à 10 bar environ et à une température de -30°C peut être réchauffé dans un échangeur de chaleur 28 disposé sur la ligne 12 en aval de l'ajout 42 de méthanol, par échange direct avec la charge liquide d'isobutane, et peut contribuer de ce fait au sous-refroidissement de la charge avant sa vaporisation.

En fond du séparateur, on récupère par une ligne 18 une phase liquide hydrocarbonée à -25°C et 15 bar absolus environ, contenant au moins 95 % d'isobutène, qui refroidit dans un échangeur de chaleur 19 l'effluent avant son entrée dans l'échangeur de chaleur réfrigérant 13. La phase liquide hydrocarbonée est à nouveau réchauffée dans un autre échangeur de chaleur 21 avant d'être introduite dans une colonne de stabilisation 20 délivrant en tête du fuel gaz par une ligne 33 et en fond par une ligne 35 de l'isobutène et de l'isobutane non converti qui contribuent à l'échange thermique dans l'échangeur 21 et que l'on envoie dans une unité de formation de MTBE (non représentée).

On peut contrôler la température du séparateur en ajustant le niveau de pression dans la calandre de l'échangeur 13 soit par l'intermédiaire d'une vanne sur la ligne 2 en sortie de l'échangeur, soit par le contrôle de la vitesse du compresseur 14.

## Revendications

1. Procédé de déshydrogénation catalytique d'une charge hydrocarbonée paraffinique C₂+ comprenant une étape de déshydrogénation dans une zone de déshydrogénation de ladite charge en phase gazeuse et éventuellement en présence d'hydrogène délivrant un effluent de déshydrogénation comprenant de l'eau, de l'hydrogène, des hydrocarbures oléfiniques et des hydrocarbures paraffiniques non convertis, au moins une étape de refroidissement de l'effluent dans une zone de refroidissement indirect avec ladite charge, une étape de compression de l'effluent à une pression appropriée, une étape d'élimination de l'eau de l'effluent comprimé, une étape de réfrigération dans un échangeur de chaleur à double enceinte de l'effluent comprimé, une étape de séparation de l'effluent réfrigéré et une étape de récupération d'une part d'une phase vapeur riche en hydrogène et d'autre part d'une phase liquide hydrocarbonée comprenant les hydrocarbures oléfiniques et les hydrocarbures non convertis, le procédé étant caractérisé en ce que l'étape de réfrigération comprend l'introduction dans une première enceinte dudit échangeur de chaleur de la charge hydrocarbonée en phase liquide, I'échangeur de chaleur étant adapté à vaporiser ladite charge dans la première enceinte et à réfrigérer par échange indirect l'effluent comprimé dans la seconde enceinte, le procédé étant en outre caractérisé en ce qu'on récupère de la première enceinte de l'échangeur de chaleur la charge vaporisée que l'on introduit dans la zone de déshydrogénation.

2. Procédé selon la revendication 1 dans lequel on recycle une partie au moins de la phase riche en hydrogène dans la première enceinte de l'échangeur de chaleur où on la met en contact avec la charge liquide et on récupère la charge vaporisée contenant l'hydrogène que l'on introduit dans la zone de déshydrogénation.

3. Procédé selon la revendication 1 dans lequel on détend de manière isentropique par des moyens de détente appropriés la phase vapeur riche en hydrogène de façon à séparer un condensat résiduel hydrocarboné et de I'hydrogène, on recycle en partie au moins l'hydrogène ainsi séparé dans la première enceinte de l'échangeur de chaleur où on le met en contact avec la charge liquide et on récupère la charge vaporisée et l'hydrogène que l'on introduit dans la zone de déshydrogénation.

4. Procédé selon la revendication 3 dans lequel le condensat résiduel hydrocarboné est mélangé à l'effluent sortant de la seconde enceinte de l'échangeur de chaleur.

5. Procédé selon l'une des revendications 1 à 4 dans lequel ladite charge comprend des hydrocarbures paraffiniques à 3, 4 ou/et 5 atomes de carbone.

6. Procédé selon l'une des revendications 1 à 5 dans lequel ladite charge comprend essentiellement de l'isobutane.

7. Procédé selon l'une des revendications 1 à 6 dans lequel l'étape de compression de l'effluent est réalisée à une pression de 3 à 35 bars absolus.

8. Procédé selon l'une des revendications 1 à 7 dans lequel la température dans la première enceinte de l'échangeur de chaleur est de -5 à -90°C sous une pression de 1,2 à 5,0 bar absolus.

9. Procédé selon l'une des revendications 3 à 8 dans lequel la phase vapeur riche en hydrogène est détendue à une pression de 1,2 à 5,0 bar absolus sous une température de -50 à -100°C.

10. Procédé selon l'une des revendications 1 à 9 dans lequel on comprime la charge contenant éventuellement l'hydrogène, sortant de la première enceinte de l'échangeur de chaleur de façon que la pression dans ladite première enceinte de l'échangeur de chaleur où est vaporisée la charge soit de 0,2 à 3 bar et on envoie la charge vaporisée comprimée vers la zone de déshydrogénation via la zone de refroidissement.

11. Procédé selon l'une des revendications 3 à 10 dans lequel l'autre partie de l'hydrogène séparé du condensat est comprimé à une pression de 5 à 10 bar absolus par un compresseur mis en mouvement par les moyens de détente de façon à refroidir ladite charge liquide.

12. Unité de déshydrogénation catalytique comprenant en combinaison un réacteur de déshydrogénation (40), des moyens d'alimentation en une charge gazeuse hydrocarbonée et éventuellement des moyens d'alimentation en hydrogène, connectés à une entrée de réacteur (40), au moins un moyen (41) de refroidissement d'un effluent relié à une sortie du réacteur, des moyens de compression (6) de l'effluent connectés aux moyens de refroidissement, des moyens d'élimination (10) de l'eau contenue dans l'effluent connectés aux moyens de compression, des moyens de réfrigération (13) de l'effluent comprimé reliés aux moyens d'élimination de l'eau, des moyens de séparation (8) de l'effluent réfrigéré reliés aux moyens de réfrigération, des moyens de récupération (22) d'une phase riche en hydrogène et des moyens de récupération (18) d'une phase riche en hydrocarbures oléfiniques, ladite unité étant caractérisée en ce que les moyens de réfrigération comprennent un échangeur (13) indirect de chaleur à double enceinte, une première enceinte ayant une entrée reliée aux moyens d'alimentation (12) en la charge liquide et une sortie reliée aux moyens de refroidissement (41), ledit échangeur (13) de chaleur étant adapté à vaporiser la charge dans ladite première enceinte, à l'évacuer par ladite sortie et à réfrigérer l'effluent par échange indirect de chaleur dans la seconde enceinte, ladite seconde enceinte étant reliée aux moyens de séparation (8).

13. Unité selon la revendication 12 dans laquelle la première enceinte de l'échangeur de chaleur (13) comporte une autre entrée connectée aux moyens de récupération d'une phase riche en hydrogène (15) et des moyens adaptés à mélanger l'hydrogène et la charge.

14. Unité selon la revendication 13 dans laquelle les moyens de récupération d'une phase riche en hydrogène (22) comprennent des moyens de détente (23) connectés à un séparateur (27) de phases, ledit séparateur de phases ayant une première sortie de gaz hydrogène reliée à l'autre entrée de la première enceinte de l'échangeur de chaleur.

15. Unité selon la revendication 14 dans laquelle ledit séparateur de phases (27) comporte une deuxième sortie à une extrémité opposée délivrant un condensat cryogénique reliée aux moyens de séparation (8) de l'effluent réfrigéré.

16. Unité selon l'une des revendications 12 à 15 dans laquelle un compresseur (14) est interposé entre la sortie de la première enceinte de l'échangeur de chaleur et les moyens de refroidissement (41) de l'effluent.

## Patentansprüche

1. Verfahren zur katalytischen Dehydrierung einer paraffinischen C₂₊-Kohlenwasserstoffcharge, umfassend einen Schritt zur Dehydrierung der in Gasphase vorliegenden Charge in einer Dehydrierungszone und gegebenenfalls in Anwesenheit von Wasserstoff, die einen Dehydrierungsabstrom liefert, welcher Wasser, Wasserstoff, olefinische Kohlenwasserstoffe und nicht umgewandelte paraffinische Kohlenwasserstoffe umfaßt, wenigstens einen Schritt zur Abkühlung des Abstromes in einer Zone zur indirekten Abkühlung mit der Charge, einen Schritt zur Komprimierung des Abstromes auf einen geeigneten Druck, einen Schritt zur Entfernung von Wasser aus dem komprimierten Abstrom, einen Schritt zur Kühlung des komprimierten Abstromes in einem doppelwandigen Wärmetauscher, einen Schritt zur Trennung des gekühlten Abstromes und einen Schritt zur Gewinnung einer an Wasserstoff reichen Dampfphase einerseits und einer kohlenwasserstoffhaltigen Flüssigphase andererseits, welche die olefinischen Kohlenwasserstoffe und die nicht umgewandelten Kohlenwasserstoffe umfaßt, wobei das Verfahren dadurch gekennzeichnet ist, daß der Schritt zur Kühlung die Einführung der in Flüssigphase vorliegenden Kohlenwasserstoffphase in einen ersten Raum des Wärmetauschers umfaßt, wobei der Wärmetauscher geeignet ist, die Charge in dem ersten Raum zu verdampfen und den komprimierten Abstrom in dem zweiten Raum durch einen indirekten Austausch zu kühlen, wobei das Verfahren außerdem dadurch gekennzeichnet ist, daß man die verdampfte Charge aus dem ersten Raum des Wärmetauschers gewinnt, die man in die Dehydrierungszone einführt.

2. Verfahren nach Anspruch 1, bei welchem man einen Teil wenigstens der an Wasserstoff reichen Phase in den ersten Raum des Wärmetauschers zurückführt, in welchem man sie mit der flüssigen Charge in Kontakt bringt, und man die Wasserstoff enthaltende verdampfte Charge gewinnt, die man in die Dehydrierungszone einführt.

3. Verfahren nach Anspruch 1, bei welchem man die an Wasserstoff reiche Dampfphase durch geeignete Entspannungseinrichtungen auf isentropische Weise entspannt, derart, um ein kohlenwasserstoffhaltiges Rückstandskondensat und den Wasserstoff zu trennen, man teilweise wenigstens den so getrennten Wasserstoff in den ersten Raum des Wärmetauschers zurückführt, in welchem man ihn mit der flüssigen Charge in Kontakt bringt, und man die verdampfte Charge und den Wasserstoff, die man in die Dehydrierungszone einführt, wiedergewinnt.

4. Verfahren nach Anspruch 3, bei welchem das kohlenwasserstoffhaltige Rückstandskondensat mit einem Abstrom gemischt wird, der den zweiten Raum des Wärmetauschers verläßt.

5. Verfahren nach einem der Ansprüche 1 bis 4, bei welchem die Charge paraffinische Kohlenwasserstoffe mit 3, 4 und/oder 5 Kohlenstoffatomen umfaßt.

6. Verfahren nach einem der Ansprüche 1 bis 5, bei welchem die Charge im wesentlichen Isobutan umfaßt.

7. Verfahren nach einem der Ansprüche 1 bis 6, bei welchem der Schritt zur Komprimierung des Abstromes bei einem Absolutdruck von 3 bis 35 bar durchgeführt wird.

8. Verfahren nach einem der Ansprüche 1 bis 7, bei welchem die Temperatur in dem ersten Raum des Wärmetauschers von -5 bis -90 °C bei einem Absolutdruck von 1,2 bis 5,0 bar beträgt.

9. Verfahren nach einem der Ansprüche 3 bis 8, bei welchem die an Wasserstoff reiche Dampfphase auf einen Absolutdruck von 1,2 bis 5,0 bar bei einer Temperatur von -50 bis -100 °C entspannt wird.

10. Verfahren nach einem der Ansprüche 1 bis 9, bei welchem man die gegebenenfalls Wasserstoff enthaltende Charge, die den ersten Raum des Wärmetauschers verläßt, komprimiert, derart, daß der Druck in dem ersten Raum des Wärmetauschers, in welchem die Charge verdampft wird, von 0,2 bis 3 bar beträgt, und man der Dehydrierungszone die komprimierte verdampfte Charge über die Abkühlungszone zuführt.

11. Verfahren nach einem der Ansprüche 3 bis 10, bei welchem der andere Teil des Wasserstoffs, der von dem Kondensat abgetrennt ist, auf einen Absolutdruck von 5 bis 10 bar durch einen Verdichter komprimiert wird, welcher durch die Entspannungseinrichtungen angetrieben ist, derart, um die flüssige Charge abzukühlen.

12. Einheit zur katalytischen Dehydrierung, in Kombination umfassend einen Reaktor (40) zur Dehydrierung, Einrichtungen zur Einspeisung einer gasförmigen Kohlenwasserstoffcharge und gegebenenfalls Einrichtungen zur Einspeisung von Wasserstoff, die mit einem Einlaß des Reaktors (40) verbunden sind, wenigstens eine Einrichtung (41) zur Abkühlung eines Abstromes, die mit einem Auslaß des Reaktors verbunden ist, Einrichtungen (6) zur Komprimierung des Abstromes, die mit den Einrichtungen zur Abkühlung verbunden sind, Einrichtungen (10) zur Entfernung von in dem Abstrom enthaltenem Wasser, die mit den Einrichtungen zur Komprimierung verbunden sind, Einrichtungen (13) zur Kühlung des komprimierten Abstromes, die mit den Einrichtungen zur Entfernung des Wassers verbunden sind, Einrichtungen (8) zur Trennung des gekühlten Abstromes, die mit den Einrichtungen zur Kühlung verbunden sind, Einrichtungen (22) zur Gewinnung einer an Wasserstoff reichen Phase und Einrichtungen (18) zur Gewinnung einer an olefinischen Kohlenwasserstoffen reichen Phase, wobei die Einheit dadurch gekennzeichnet ist, daß die Einrichtungen zur Abkühlung einen doppelwandigen, indirekten Wärmetauscher (13) umfassen, wobei ein erster Raum einen Einlaß, der mit den Einrichtungen (12) zur Zuführung der flüssigen Charge verbunden ist, und einen Auslaß, der mit den Einrichtungen (41) zur Abkühlung verbunden ist, aufweist, wobei der Wärmetauscher (13) geeignet ist, die Charge in dem ersten Raum zu verdampfen, sie durch den Auslaß zu evakuieren und den Abstrom durch indirekten Wärmeaustausch in dem zweiten Raum zu kühlen, wobei der zweite Raum mit den Einrichtungen (8) zur Trennung verbunden ist.

13. Einheit nach Anspruch 12, bei welcher der erste Raum des Wärmetauschers (13) einen weiteren Einlaß, der mit Einrichtungen (15) zur Gewinnung einer an Wasserstoff reichen Phase verbunden ist, und Einrichtungen, die geeignet sind, den Wasserstoff und die Charge zu mischen, umfaßt.

14. Einheit nach Anspruch 13, bei welcher die Einrichtungen (22) zur Gewinnung einer an Wasserstoff reichen Phase Einrichtungen (23) zur Entspannung umfassen, die mit einem Phasenabscheider (27) verbunden sind, wobei der Phasenabscheider einen ersten Auslaß für Wasserstoffgas aufweist, der mit dem weiteren Einlaß des ersten Raumes des Wärmetauschers verbunden ist.

15. Einheit nach Anspruch 14, bei welcher der Phasenabscheider (27) einen zweiten Auslaß an einem gegenüberliegenden Ende umfaßt, der ein Tiefsttemperaturkondensat liefert, welcher mit den Einrichtungen (8) zur Trennung des gekühlten Abstromes verbunden ist.

16. Einheit nach einem der Ansprüche 12 bis 15, bei welcher ein Verdichter (14) zwischen dem Auslaß des ersten Raumes des Wärmetauschers und den Einrichtungen (41) zur Abkühlung des Abstromes angeordnet ist.

## Claims

1. Process of catalytic dehydrogenation of a C₂₊ paraffinic hydrocarbon charge comprising a dehydrogenation step in a dehydrogenation zone of said charge in gas phase and optionally in the presence of hydrogen delivering a dehydrogenation effluent comprising water, hydrogen, olefinic hydrocarbons and unconverted paraffinic hydrocarbons, at least one step of cooling the effluent in an indirect cooling zone with said charge, a step of compressing the effluent at a suitable pressure, a step of eliminating water of the compressed effluent, a step of cooling the compressed effluent in a dual-chamber heat exchanger, a step of separating the cooled effluent and a step of recovering, on the one hand, a hydrogen-rich vapor phase and, on the other hand, a hydrocarbon liquid phase comprising olefinic hydrocarbons and unconverted hydrocarbons, the process being characterized in that the cooling step comprises the introduction, into a first chamber of said heat exchanger, of the hydrocarbon charge in liquid phase, the heat exchanger being suited to evaporate said charge in the first chamber and to cool, by indirect exchange, the compressed effluent in the second chamber, the process further being characterized in that the evaporated charge that is introduced into the dehydrogenation zone is recovered from the first chamber of the heat exchanger.

2. Process according to claim 1, wherein at least one part of the hydrogen-rich phase is recycled in the first chamber of the heat exchanger where it is put in contact with the liquid charge and the evaporated charge containing the hydrogen that is introduced into the dehydrogenation zone is recovered.

3. Process according to claim 1, wherein the hydrogen-rich vapor phase is expanded isentropically by suitable expansion means to separate a hydrocarbon residual condensate and hydrogen, the hydrogen thus separated is recycled at least partly in the first chamber of the heat exchanger where it is put in contact with the liquid charge and the evaporated charge and the hydrogen that are introduced into the dehydrogenation zone are recovered.

4. Process according to claim 3, wherein the hydrocarbon residual condensate is mixed with the effluent going out from the second chamber of the heat exchanger.

5. Process according to one of claims 1 to 4, wherein said charge comprises paraffinic hydrocarbons with 3, 4 and/or 5 carbon atoms.

6. Process according to one of claims 1 to 5, wherein said charge essentially comprises isobutane.

7. Process according to one of claims 1 to 6, wherein the step of compressing the effluent is performed at a pressure of 3 to 35 absolute bars.

8. Process according to one of claims 1 to 7, wherein the temperature in the first chamber of the heat exchanger is -5 to -90°C under a pressure of 1.2 to 5.0 absolute bars.

9. Process according to one of claims 3 to 8, wherein the hydrogen-rich vapor phase is expanded at a pressure of 1.2 to 5.0 absolute bars under a temperature of -50 to -100°C.

10. Process according to one of claims 1 to 9, wherein the charge optionally containing hydrogen is compressed, going out of the first chamber of the heat exchanger so that the pressure in said first chamber of the heat exchanger where the charge is evaporated is 0.2 to 3 bars, and the compressed, evaporated charge is sent to the dehydrogenation zone via the cooling zone.

11. Process according to one of claims 3 to 10, wherein the other part of the hydrogen separated from the condensate is compressed at a pressure of 5 to 10 absolute bars by a compressor set in motion by the expansion means to cool said liquid charge.

12. Catalytic dehydrogenation unit comprising in combination a dehydrogenation reactor (40), means for feeding a hydrocarbon gas charge and optionally means for feeding hydrogen, connected to a reactor input (40), at least one means (41) for cooling an effluent connected to an output of the reactor, means (6) for compressing the effluent connected to the cooling means, means (10) for eliminating water contained in the effluent connected to the compression means, means (13) for cooling the compressed effluent connected to the means for eliminating water, means (8) for separating the cooled effluent connected to the cooling means, means (22) for recovering a hydrogen-rich phase and means (18) for recovering an olefinic hydrocarbon-rich phase, said unit being characterized in that the cooling means comprise a dual-chamber indirect heat exchanger (13), a first chamber having -an input connected to means (12) for feeding the liquid charge and an output connected to cooling means (41), said heat exchanger (13) being suited to evaporate the charge in said first chamber, to evacuate it by said output and to cool the effluent by indirect heat exchange in the second chamber, said second chamber being connected to separating means (8).

13. Unit according to claim 12, wherein the first chamber of heat exchanger (13) comprises another input connected to the means for recovering a hydrogen-rich phase (15) and means suited to mixing hydrogen and the charge.

14. Unit according to claim 13, wherein the means for recovering a hydrogen-rich phase (22) comprise expansion means (23) connected to a phase separator (27), said phase separator having a first hydrogen gas output connected to the other input of the first chamber of the heat exchanger.

15. Unit according to claim 14, wherein said phase separator (27) comprises a second output at an opposite end delivering a cryogenic condensate connected to means (8) for separating the cooled effluent.

16. Unit according to one of claims 12 to 15, wherein a compressor (14) is inserted between the output of the first chamber of the heat exchanger and means (41) for cooling the effluent.
